# EUROPEAN PATENT SPECIFICATION

(11) **EP 1 072 270 B1**
(45) Date of publication and mention of the grant of the patent: **19.10.2005**
(21) Application number: 00203294.4
(22) Date of filing: 16.12.1994
(51) Int. Cl.: A61K 38/48, A61K 38/16, A61P 21/02

(54) **Botulinum toxin for treating dystonia**
Botulinumtoxin zur Behandlung von Dystonie
Toxine de Botulinum pour le traitement de la dystonie

(30) Priority: 28.12.1993 US 173996
(43) Date of publication of application: 31.01.2001
(62) Divisional of application: 95906674.7
(73) Proprietor: ALLERGAN, INC., Irvine, CA 92612 (US)
(72) Inventor: Aoki, K. Roger, Coto de Caza, CA 92679 (US); Grayston, Michael W., Irvine, CA 92714 (US); Carlson, Steven R., San Mateo, CA 94402 (US); Leon, Judith M., Laguna Niguel, CA 92677 (US)
(74) Representative: Frank, Veit Peter

(56) References cited:
- WO-A-94/00481
- WO-A-94/28922
- WO-A-94/28923
- DATABASE PHIN [Online] STN; Database accession no. 92:5718 XP002199876 & "Athena Neurosciences' strategic plan" SCRIP (NEWSLETTER), no. 1711, 22 April 1992 (1992-04-22), page 15
- DATABASE PHIN [Online] STN; Database accession no. 92:6599 XP002199877 & "Athena files US and Canadian Investigational New Drugs (INDs) for AN-072" SCRIP (NEWSLETTER), no. 1724, 5 June 1992 (1992-06-05), page 29
- DATABASE NLDB [Online] STN; Database accession no. 93:176963 XP002199878 & GENESIS GROUP ASSOCIATES INC.: "Focal Dystonia: In Phase 1/II Clinicals" GENESIS REPORT-RX (NEWSLETTER), vol. 2, no. 2, February 1993 (1993-02),
- DATABASE NLDB [Online] STN; Database accession no. 92:332309 XP002199879 & "Athena outlines business strategy" MARKETLETTER (NEWSLETTER), 5 October 1992 (1992-10-05),
- SCHANTZ E J ET AL: "PROPERTIES AND USE OF BOTULINUM TOXIN AND OTHER MICROBIAL NEUROTOXINS IN MEDICINE" MICROBIOLOGICAL REVIEWS, AMERICAN SOCIETY FOR MICROBIOLOGY, WASHINGTON, DC, US, vol. 56, no. 1, 1 March 1992 (1992-03-01), pages 80-99, XP000569909 ISSN: 0146-0749

## Description

The present invention provides treatments for dystonia using botulinum toxin type B.

Heretofore, Botulinum toxins, in particular Botulinum toxin type A, has been used in the treatment of a number of neuromuscular disorders and conditions involving muscular spasm; for example, strabismus, blepharospasm, spasmodic torticollis (cervical dystonia), oromandibular dystonia and spasmodic dysphonia (laryngeal dystonia). The toxin binds rapidly and strongly to presynaptic cholinergic nerve terminals and inhibits the exocytosis of acetylcholine by decreasing the frequency of acetylcholine release. This results in local paralysis and hence relaxation of the muscle afflicted by spasm.

For one example of treating neuromuscular disorders, see U.S. Patent No. 5,053,005 to Borodic, which suggests treating curvature of the juvenile spine, i.e., scoliosis, with an acetylcholine release inhibitor, preferably Botulinum toxin A.

For the treatment of strabismus with Botulinum toxin type A, see Elston, J.S., et al., *British Journal of Ophthalmology,* 1985, 69, 718-724 and 891-896. For the treatment of blepharospasm with Botulinum toxin type A, see Adenis, J.P., et al., *J. Fr. Ophthalmol.,* 1990, 13 (5) at pages 259-264. For treating squint, see Elston, J.S., *Eye,* 1990, 4(4):VII. For treating spasmodic and oromandibular dystonia torticollis, see Jankovic et al., *Neurology,* 1987, 37, 616-623.

Spasmodic dysphonia has been treated with Botulinum toxin type A. See Blitzer et al., *Ann. Otol. Rhino. Laryngol,* 1985, 94, 591-594. Lingual dystonia was treated with Botulinum toxin type A according to Brin et al., *Adv. Neurol.* (1987) 50, 599-608. Finally, Cohen et al., *Neurology* (1987) 37 (Suppl. 1), 123-4, discloses the treatment of writer's cramp with Botulinum toxin type A.

The term Botulinum toxin is a generic term embracing the family of toxins produced by the anaerobic bacterium *Clostridium botulinum* and, to date, seven immunologically distinct neurotoxins have been identified. These have been given the designations A, B, C, D, E, F and G. For further information concerning the properties of the various Botulinum toxins, reference is made to the article by Jankovic and Brin, *The New England Journal of Medicine,* No. 17, 1990, pp. 1186-1194, and to the review by Charles L. Hatheway in Chapter 1 of the book entitled *Botulinum Neurotoxin and Tetanus Toxin,* L. L. Simpson, Ed., published by Academic Press Inc. of San Diego, California, 1989.

Scrip (1992), no. 1711, p15; Scrip (1992) no. 1724, p29; Genesis Report (Feb 1993) vol. 2, no. 2 and Athena Marketletter of October 5, 1992 contemplate the use of a botulinum toxin type B fragment for treating various disorders including spasmodic torticollis.

The neurotoxic component of Botulinum toxin has a molecular weight of about 150 kilodaltons and is thought to comprise a short polypeptide chain of about 50 kD which is considered to be responsible for the toxic properties of the toxin, i.e., by interfering with the exocytosis of acetylcholine, by decreasing the frequency of acetylcholine release, and a larger polypeptide chain of about 100 kD which is believed to be necessary to enable the toxin to bind to the presynaptic membrane.

The "short" and "long" chains are linked together by means of a simple disulfide bridge. (It is noted that certain serotypes of Botulinum toxin, e.g., type E, may exist in the form of a single chain un-nicked protein, as opposed to a dichain. The single chain form is less active but may be converted to the corresponding dichain by nicking with a protease, e.g., trypsin. Both the single and the dichain are useful in the method of the present invention.)

In general, four physiologic groups of *C. botulinum* are recognized (I, II, III, IV). The organisms capable of producing a serologically distinct toxin may come from more than one physiological group. For example, Type B and F toxins can be produced by strains from Group I or II. In addition, other strains of clostridial species (*C. baratii,* type F; *C. butyricum,* type E; *C. novyi,* type C₁ or D) have been identified which can produce botulinum neurotoxins.

Immunotoxin conjugates of ricin and antibodies, which are characterized as having enhanced cytotoxicity through improving cell surface affinity, are disclosed in European Patent Specification 0 129 434. The inventors note that botulinum toxin may be utilized in place of ricin.

Botulinum toxin is obtained commercially by establishing and growing cultures of *C. botulinum* in a fermenter and then harvesting and purifying the fermented mixture in accordance with known techniques.

Botulinum toxin type A, the toxin type generally utilized in treating neuromuscular conditions, is currently available commercially from several sources; for example, from Porton Products Ltd. UK, under the trade name "DYSPORT," and from Allergan, Inc., Irvine, California, under the trade name BOTOX®.

The present invention is set forth in the claims.

Each serotype of Botulinum toxin has been identified as immunologically different proteins through the use of specific antibodies. For example, if the antibody (antitoxin) recognizes, that is, neutralizes the biological activity of, for example, type A it will not recognize types B, C, D, E, F or G.

While all of the Botulinum toxins appear to be zinc endopeptidases, the mechanism of action of different serotypes, for example, A and E within the neuron appear to be different than that of Type B. In addition, the neuronal surface "receptor" for the toxin appears to be different for the serotypes.

In the area of use of the Botulinum toxins in accordance with the present invention with regard to organ systems which involve the release of neurotransmitter, it is expected to introduce the toxins B, directly by local injections.

The Botulinum toxin used according to the present invention is Botulinum toxin type B.

The physiologic groups of *Clostridium botulinum* types are listed in Table I.

**Table I**

| Physiologic Groups or *Clostridium botulinum* | | | | | | | |
|---|---|---|---|---|---|---|---|
| Group | Toxin Sero-Type | Biochemistry | Milk Digest | Glucose Fermentation | Lipase | Phages & Plasmids | Phenotypically Related Clostridium (nontoxigenic) |
| I | A,B,F | proteolytic saccharolytic | + | + | + | + | C. sporogenes |
| II | B,E,F | nonproteolytic saccharolytic psychotropic | - | + | + | + | |
| III | C,D | nonproteolytic saccharolytic | + | + | + | + | C. novyi |
| IV | G | proteolytic nonsaccharolytic | + | - | - | - | C. subterminale |

These toxin types may be produced by selection from the appropriate physiologic group of *Clostridium botulinum* organisms. the organisms designated as Group I are usually referred to as proteolytic and produce Botulinum toxins of types A, B and F. The organisms designated as Group II are saccharolytic and produce Botulinum toxins of types B, E and F. The organisms designated as Group III produce only Botulinum toxin types C and D and are distinguished from organisms of Groups I and II by the production of significant amounts of propionic acid. Group IV organisms only produce neurotoxin of type G. The production of any and all of the Botulinum toxin types A, B, C, D, E, F and G are described in Chapter 1 of *Botulinum Neurotoxin and Tetanus Toxin,* cited above, and/or the references cited therein. Botulinum toxins types B, C, D, E, F and G are also available from various species of clostridia.

Currently fourteen species of clostridia are considered pathogenic. Most of the pathogenic strains produce toxins which are responsible for the various pathological signs and symptoms. Organisms which produce Botulinum toxins have been isolated from botulism outbreaks in humans (types A, B, E and F) and animals (types C and D). Their identities were described through the use of specific antitoxins (antibodies) developed against the earlier toxins. Type G toxin was found in soil and has low toxigenicity. However, it has been isolated from autopsy specimens, but thus far there has not been adequate evidence that type G botulism has occurred in humans.

Preferably, the toxin is administered by means of intramuscular injection directly into a local area such as a spastic muscle, preferably in the region of the neuromuscular junction, although alternative types of administration (e.g., subcutaneous injection), which can deliver the toxin directly to the affected region, may be employed where appropriate. The toxin can be presented as a sterile pyrogen-free aqueous solution or dispersion and as a sterile powder for reconstitution into a sterile solution or dispersion.

Where desired, tonicity adjusting agents such as sodium chloride, glycerol and various sugars can be added. Stabilizers such as human serum albumin may also be included. The formulation may be preserved by means of a suitable pharmaceutically acceptable preservative such as a paraben, although preferably it is unpreserved.

It is preferred that the toxin is formulated in unit dosage form; for example, it can be provided as a sterile solution in a vial or as a vial or sachet containing a lyophilized powder for reconstituting a suitable vehicle such as saline for injection.

In one embodiment, the Botulinum toxin is formulated in a solution containing saline and pasteurized human serum albumin, which stabilizes the toxin and minimizes loss through non-specific adsorption. The solution is sterile filtered (0.2 micron filter), filled into individual vials and then vacuum-dried to give a sterile lyophilized powder. In use, the powder can be reconstituted by the addition of sterile unpreserved normal saline (sodium chloride 0.9% for injection).

The dose of toxin administered to the patient will depend upon the severity of the condition; e.g., the number of muscle groups requiring treatment, the age and size of the patient and the potency of the toxin. The potency of the toxin is expressed as a multiple of the LD₅₀ value for the mouse, one unit (U) of toxin being defined as being the equivalent amount of toxin that kills 50% of a group of 18 to 20 female Swiss-Webster mice, weighing about 20 grams each.

The dosages used in human therapeutic applications are roughly proportional to the mass of muscle being injected. Typically, the dose administered to the patient may be up from about 0.01 to about 1,000 units; for example, up to about 500 units, and preferably in the range from about 80 to about 460 units per patient per treatment, although smaller of larger doses may be administered in appropriate circumstances such as up to about 50 units for the relief of pain and in controlling cholinergic secretions.

As the physicians become more familiar with the use of this product, the dose may be changed. The potency of Botulinum toxin and its long duration of action mean that doses will tend to be administered on an infrequent basis. Ultimately, however, both the quantity of toxin administered and the frequency of its administration will be at the discretion of the physician responsible for the treatment and will be commensurate with questions of safety and the effects produced by the toxin.

The invention will now be illustrated by reference to the following nonlimiting examples.

In each of the examples, appropriate areas of each patient are injected with a sterile solution containing the confirmation of Botulinum toxin. Total patient doses range from about 0.01 units to 460 units. Before injecting any muscle group, careful consideration is given to the anatomy of the muscle group, the aim being to inject the area with the highest concentration of neuromuscular junctions, if known. Before injecting the muscle, the position of the needle in the muscle is confirmed by putting the muscle through its range of motion and observing the resultant motion of the needle end. General anaesthesia, local anaesthesia and sedation are used according to the age of the patient, the number of sites to be injected, and the particular needs of the patient. More than one injection and/or sites of injection may be necessary to achieve the desired result. Also, some injections, depending on the muscle to be injected, may require the use of fine, hollow, teflon-coated needles, guided by electromyography.

Following injection, it is noted that there are no systemic or local side effects and none of the patients are found to develop extensive local hypotonicity. The majority of patients show an improvement in function both subjectively and when measured objectively.

### Example 1

### The Use of Botulinum toxin Type in the Treatment of Tardive Dyskinesia

A male patient, age 45, suffering from tardive dyskinesia resulting from the treatment with an antipsychotic drug, such as Thorazine or Haldol, is treated with 150 units of Botulinum toxin type B by direct injection of such toxin into the facial muscles. After 1-3 days, the symptoms of tardive dyskinesia, i.e., orofacial dyskinesia, athetosis, dystonia, chorea, tics and facial grimacing, etc. are markedly reduced.

### Example 2

### The Use of Botulinum toxin Type B in the Treatment of Spasmodic Torticollis

A patient suffering from spasmodic torticollis, as manifested by spasmodic or tonic contractions of the neck musculature, producing stereotyped abnormal deviations of the head, the chin being rotated to one side, and the shoulder being elevated toward the side at which the head is rotated, is treated by injection with 100-1,000 units of Botulinum toxin type B. After 3-7 days, the symptoms are substantially alleviated; i.e., the patient is able to hold his head and shoulder in a normal position.

### Example 3

### The Use of Botulinum toxin in the Treatment of Essential Tremor

A male, age 45, suffering from essential tremor, which is manifested as a rhythmical oscillation of head or hand muscles and is provoked by maintenance of posture or movement, is treated by injection with 50-1,000 units of Botulinum toxin type B. After two to eight weeks, the symptoms are substantially alleviated; i.e., the patient's head or hand ceases to oscillate.

### Example 4

### The Use of Botulinum toxin in the Treatment of Spasmodic Dysphonia

A male, age 45, unable to speak clearly, due to spasm of the vocal chords, is treated by injection of the vocal chords with Botulinum toxin type B, having an activity of 80-500 units. After 3-7 days, the patient is able to speak clearly.

## Claims

1. The use of botulinum toxin type B for the manufacture of a medicament for treating dystonia, provided that:
i) the medicament is not a frozen, dried preparation comprising C. botulinum type B neurotoxin associated with botulinum-derived stabilizing proteins to form a complex, the complex having a molecular weight as determined by gel filtration chromatography of at least 300 kD, in admixture with a pharmaceutically acceptable excipient for maintaining the stability of the complex at concentrations of 10⁴ mouse LD₅₀ units per millilitre or less, which when reconstituted in aqueous solution retains at least 75% of its toxic activity, for treating spasmodic torticollis, spasmodic dysphonia, blepharospasms and regional hand dystonias;
ii) the medicament is not a combination of botulinum toxin types A and B for treating tardive dyskinesia, essential tremor, spasmodic dysphonia or blepharospasm; and
iii) the medicament is not for treating tardive dyskinesia subsequent to a treatment with botulinum toxin type A or spasmodic torticollis subsequent to a treatment with botulinum type E.

2. The use according to claim 1 wherein the dystonia is tardive dyskinesia.

3. The use according to claim 1 wherein the dystonia is spasmodic torticollis.

4. The use according to claim 1 wherein the dystonia is essential tremor.

5. The use according to claim 1 wherein the dystonia is spasmodic dysphonia.

6. The use according to any one of the preceding claims wherein the medicament is for administration by intramuscular injection.

## Patentansprüche

1. Verwendung von Botulinumtoxin Typ B zur Herstellung eines Medikaments zur Behandlung von Dystonie, vorausgesetzt daß:
i) das Medikament kein gefrorenes, getrocknetes Präparat ist, umfassend
C. botulinum Typ B Neurotoxin assoziiert mit aus Botulinum stammenden, stabilisierenden Proteinen, um einen Komplex zu bilden, wobei der Komplex ein Molekulargewicht wie durch Gelfiltrationschromatografie bestimmt von zumindest 300 kD hat,
in Mischung mit einem pharmazeutisch annehmbaren Exzipienten zur Aufrechterhaltung der Stabilität des Komplexes bei Konzentrationen von 10⁴ Maus-LD₅₀-Einheiten pro ml oder weniger,
das, wenn es in wäßriger Lösung rekonstituiert wird, zumindest 75 % seiner toxischen Aktivität behält,
zur Behandlung von spastischem Schiefhals, spasmodischer Dysphonie, Blepharospasmus und regionalen Handdystonien;
ii) das Medikament keine Kombination der Botulinumtoxin Typen A und B zur Behandlung von tardiver Dyskinesie, essentiellem Tremor, spasmodischer Dysphonie oder Blepharospasmus ist; und
iii) das Medikament nicht zur Behandlung von tardiver Dyskinesie anschließend an eine Behandlung mit Botulinumtoxin Typ A oder von spastischem Schiefhals anschließend an eine Behandlung mit Botulinumtoxin Typ E ist.

2. Verwendung gemäß Anspruch 1, worin die Dystonie tardive Dyskinesie ist.

3. Verwendung gemäß Anspruch 1, worin die Dystonie spastischer Schiefhals ist.

4. Verwendung gemäß Anspruch 1, worin die Dystonie essentieller Tremor ist.

5. Verwendung gemäß Anspruch 1, worin die Dystonie spasmodische Dysphonie ist.

6. Verwendung gemäß einem der vorangehenden Ansprüche, worin das Medikament zur Verabreichung durch intramuskuläre Injektion ist.

## Revendications

1. Utilisation de la toxine botulinique de type B pour la fabrication d'un médicament destinée à traiter. la dystonie, à condition que :
i) le médicament ne soit pas une préparation séchée et congelée comprenant la neurotoxine de C. botulinum de type B associée à des protéines stabilisantes dérivées botulinique afin de former un complexe, le complexe ayant un poids moléculaire déterminé par chromatographie par perméation de gel d'au moins 300 kD, en mélange avec un excipient pharmaceutiquement acceptable pour maintenir la stabilité du complexe à des concentrations de 10⁴ unités LD₅₀ chez la souris par millilitre ou moins, qui quand elle est reconstituée en solution aqueuse conserve au moins 75 % de son activité toxique, destiné au traitement du torticolis spasmodique, de la dysphonie spastique, du blépharospasme et des dystonies régionales des mains ;
ii) le médicament ne soit pas une combinaison de toxines botuliniques de type A et B destinée au traitement de la dyskinésie tardive, du tremblement essentiel, de la dysphonie spastique ou du blépharospasme ; et
iii) le médicament ne soit pas destiné à traiter la dyskinésie tardive consécutive à un traitement par la toxine botulinique de type A ou le torticolis spasmodique consécutif à un traitement par le botulinum de type E.

2. Utilisation selon la revendication 1 dans laquelle la dystonie est une dyskinésie tardive.

3. Utilisation selon la revendication 1 dans laquelle la dystonie est un torticolis spasmodique.

4. Utilisation selon la revendication 1 dans laquelle la dystonie est un tremblement essentiel.

5. Utilisation selon la revendication 1 dans laquelle la dystonie est une dysphonie spastique.

6. Utilisation selon l'un quelconque des revendications précédentes dans laquelle le médicament est destiné à une administration par injection intramusculaire.
